## Europäisches Patentamt

(19) **European Patent Office**

**Office européen des brevets**

(11) Publication number: **0 300 923**
**A2**

(12) # EUROPEAN PATENT APPLICATION

(21) Application number: **88401923.3**

(22) Date of filing: **22.07.88**

(51) Int. Cl.⁴: **C 12 Q 1/68**
**C 07 H 21/00, C 12 N 15/00**

(30) Priority: **24.07.87 US 77393**

(43) Date of publication of application:
**25.01.89 Bulletin 89/04**

(84) Designated Contracting States:
**AT BE CH DE ES FR GB GR IT LI LU NL SE**

(71) Applicant: **UNIVERSITE LAVAL**
**Cité Universitaire**
**Québec, G1K 7P4 (CA)**

(72) Inventor: **Levesque, Roger**
**4385, Promenade des soeurs**
**Cap Rouge Quebec, G1Y 2K6 (CA)**

(74) Representative: **Gutmann, Ernest et al**
**S.C. Ernest Gutmann - Yves Plasseraud 67, boulevard Haussmann**
**F-75008 Paris (FR)**

(54) DNA probes for assaying B-lactamase production in gram-negative bacteria.

(57) There is provided a method for assaying the presence of a gene coding for the synthesis of β-lactamase in a targetted microorganism. This method comprises depositing and fixing on an inert support a sample containing DNA fragments in substantially single-stranded form and suspected of coding for the synthesis of β-lactamase; contacting the fixed single-stranded genetic material with a labelled probe having a nucleotide sequence of at least about 12 bases at least substantially complementary of a structural gene coding for the synthesis β-lactamase, the contacting being under hybridizing conditions at a pre-determined stringency; and detecting duplex formation on the support by means of the chosen label. There is also provided DNA probes comprising respectively 12, 15, 17 and 45 bases, these probes containing the conserved canonical aminoacid active site sequences common to β-lactamase genes OXA-2 and TEM-1, and DNA probes containing the conserved canonical aminoacid active site sequences common to the β-lactamase genes of OXA-1, PSE-1, ROB-1, OXA-3, OXA-4, ASF-2, PSE-4, CARB-3, CARB-4 and AER-1.

## Description

## DNA PROBES FOR ASSAYING B-LACTAMASE PRODUCTION IN GRAM-NEGATIVE BACTERIA

## BACKGROUND OF THE INVENTION

The most important biochemical mechanism for β-lactam resistance in gram-negative bacteria is the production of β-lactamase. This type of enzyme cleaves the β-lactam ring of penicillins and cephalosporins to give products devoid of anti-bacterial activity.

A wide variety of β-lactamases are known to be plasmid-mediated in gram-negative bacteria. In fact, more than 27 biochemically distinct β-lactamases responsible for the resistance to penicillins, cephalosporins and related β-lactam antibiotics have been identified, traditionally by isoelectric focusing. However, novel β-lactamase types have appeared in new bacterial hosts and their dissemination is facilitated by their carriage by transposons. Indeed, most if not all β-lactamase genes are part of complex multi-resistance transposable elements.

Traditionnally, bacterial resistance to antibiotics was assayed by culturing the targetted microorganism in an antibiotic containing medium. Although the efficiency of such a technique is usually quite satisfactory, it remains expensive and time consuming.

Therefore, a new technique providing faster and more economical means for assaying bacterial resistance to antibiotics would be highly desirable.

## SUMMARY OF THE INVENTION

In accordance with the present invention, there is provided a method for assaying the presence of a gene coding for the synthesis of β-lactamase in a targetted microorganism, said method comprising:

a) depositing and fixing on an inert support a sample containing DNA fragments in substantially single-stranded form and suspected of coding for the synthesis of β-lactamase;

b) contacting said fixed single stranded genetic material with a labelled probe having a nucleotide sequence of at least about 12 bases at least substantially complementary to a nucleotide sequence of a structural gene coding for the synthesis of β-lactamase, said contacting being under hybridizing conditions at a pre-determined stringency; and

c) detecting duplex formation on said support by means of said label.

There is also provided a first series of synthetic DNA probes comprising respectivelly 12, 15, 17 and 45 bases, these probes containing the conserved canonical aminoacid active site sequences common to the β-lactamase genes OXA-2 and TEM1, and a second series of DNA probes containing the conserved canonical aminoacid active site sequences common to the β-lactamase genes of OXA-1, PSE-1, ROB-1, OXA-3, OXA-4, ASF-2, PSE-4, CARB-3, CARB-4 and AER-1.

The preparation and utility of the DNA probes of the present invention will better understood by referring to the following description.

## IN THE DRAWINGS

Figure 1 represents restriction endonuclease maps of 14 recombinant plasmids containing the β-lactamase gene.

Figure 2 represents the molecular cloning and consruction of the recombinant plasmids pMON20, pMON21, and pMON22 using the R46 plasmid and pACYC184 as a cloning vehicle.

Figure 3 represents the physical maps of recombinant plasmids carrying DNA fragments used as β-lactamase (bla) gene probes.

Figure 4 represents the nucleotide and amino acid base sequence of the OXA-2 and TEM-1 synthetic oligodeoxyribonucleotide probes.

Figure 5 represents the molecular cloning and construction of the recombinant plasmids pMON34, pMON36 and pMON38 using the pMON31 plasmid containing the SHV-1 β-lactamase gene.

Figure 6 represents the molecular cloning and construction of the recombinant plasmids pMON1025, pMON1026, pMON1027, pMON1028, pMON1036, and pMON1038 using the pMK20::Tn1413 plasmid containing the CARB-4 β-lactamase gene and pACYC184 as a cloning vehicle.

Figure 7 represents plasmid pMON234 containing the PSE-2 β-lactamase gene.

Figure 8 represents the molecular cloning and construction of the recombinant plasmids pMON54, pMON55 and pMON56 using the pMON53 plasmid containing the CARB-3 β-lactamase gene.

Figure 9 represents the molecular cloning and construction of the recombinant plasmids pMON42, pMON43 and pMON44 using the pMON41 plasmid containing the OXA-5 β-lactamase gene and pACYC184 as a cloning vehicle.

Figure 10 represents the molecular cloning and construction of the recombinant plasmids pMON703, pMON705 and pMON707 using the pMON700 plasmid containing the PSE-4 β-lactamase gene.

In these drawings, abbreviations for restriction endonuclease cleavage sites are: A, Aval; B, BamHi; Bg,

BgIII; C, ClaI; E, EcoRi; H, HindIII; HC, HincII; K, KpnI; N, NruI; P, PstI; S, SalI; Sm, SmaI; X, XhoI.

Gene abbreviations are: aadA, aminoglycoside 3″(9)-adenylyltransferase; aadB, aminoglycoside 2′-adenylyltransferase; aphC, aminoglycoside 3′-phosphotransferase; bla, β-lactamase; sul, sulfonamide; Ap. Ampicillin ; Cm, Chloramphenicol , Km, Kanamycin ; Tc, Tetracycline , Spc, spectinomycin.

Other abbreviations are: Ant, antimony; Asa, arsenate; Asi, arsenite,; IR, inverted repeat; IS, insertion sequence; MucAB, enhanced mutagenesis; per, recombinase; Rep, replication, Tra, conjugal transfer.

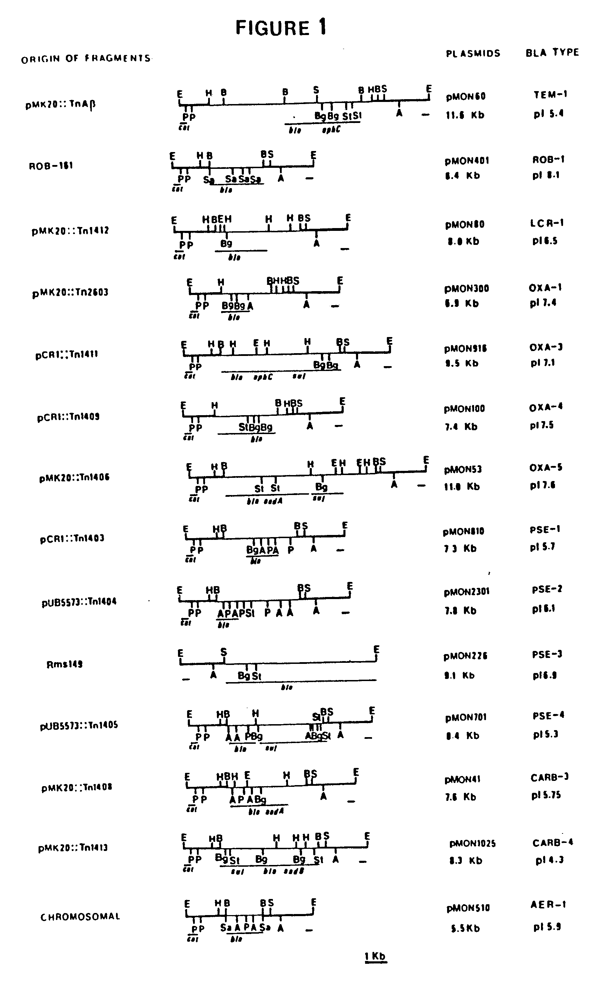

In Figure 1, plasmid maps are shown linearized at the EcoRI site of the pACYC184 vector, which is represented by a heavy line. Approximate locations of resistance determinants are also shown and recombinant molecules are drawn to scale. All 14 recombinant plasmids were mapped with the enzymes AvaI, BamHI, BgIII, EcoRI, HindIII, PvuII, SalI and SstI except that AvaI and PvuII sites were not localized for pMON401 only.

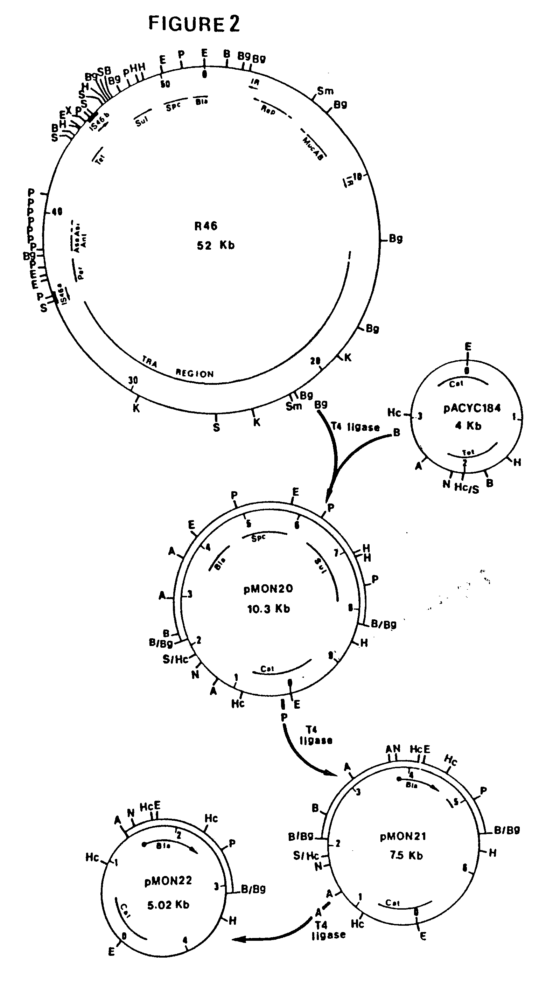

In Figure 2, coordinates are indicated in kilobases. Inverted repeat and insertion sequence orientations are indicated by arrows. The dot and arrows depicted in pMON21 and pMON22 represent the promoter location and direction of transcription for the OXA-2 bla gene.

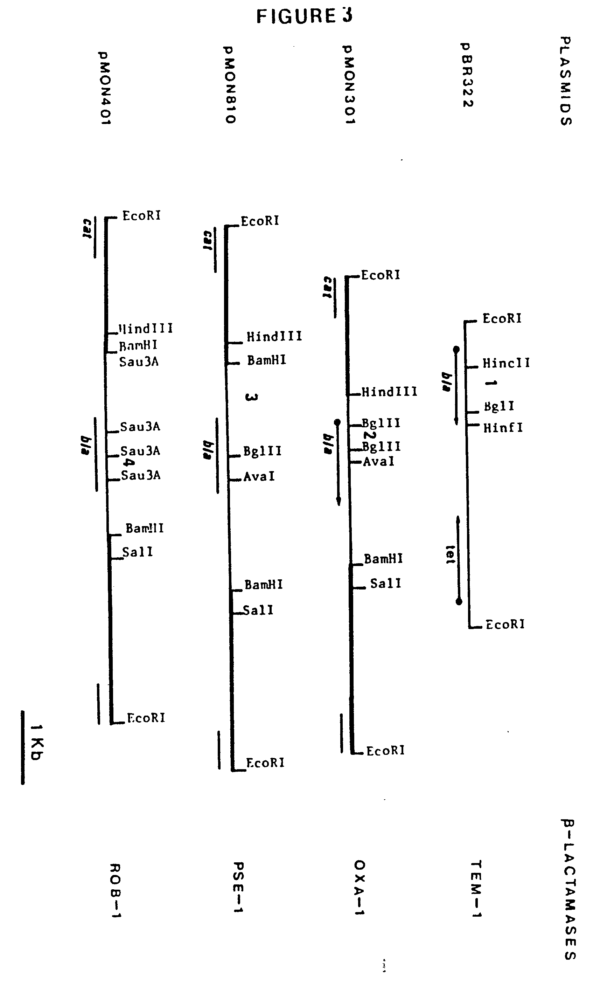

In Figure 3, only relevant endonuclease sites have been indicated. The DNA fragments used as probes are numbered 1 to 4. The thick lines represent the vector pACYC184 moiety, the dot indicates the approximate position of the promoter and the arrows indicate the direction of transcription.

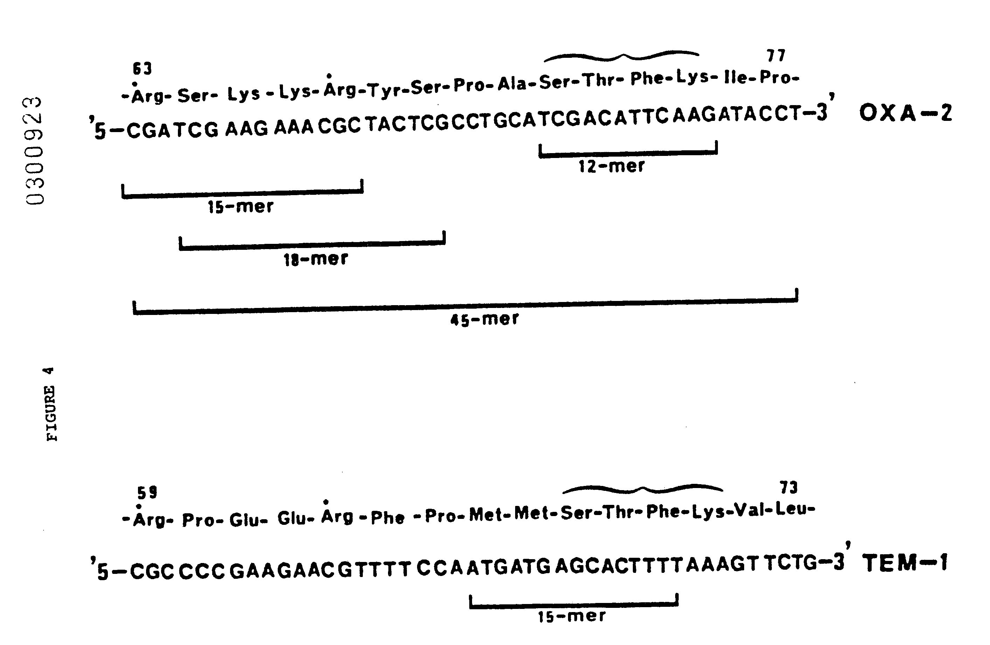

In Figure 4, the probes synthetized are delimited by heavy lines. The bracket indicates the four amino acids conserved at or near β-lactamase active sites; conserved arginine residues in both sequences are indicated by an asterisk. Location 59 to 73 for TEM-1 and 63 to 77 for OXA-2 corresponds to the position of amino acids in their respective β-lactamase structural genes.

The probes synthetized are delimited by heavy lines. The bracket indicates the four amino acids conserved at or near β-lactamase active sites; conserved arginine residues in both sequences are indicated by an asterisk. Location 59 to 73 for TEM-1 and 63 to 77 for OXA-2 corresponds to the position of amino acids in their respective β-lactamase structural genes.

## DETAILED DESCRIPTION OF THE INVENTION

Therefore, the present invention relates to a method for assaying β-lactamase production by microorganisms, leading to resistance to certain antibiotics. This method represents an improvement over existing techniques, especially in terms of rapidity, has a simple protocol, has reagents which can be standardized and provided as commercial kits, and allows for rapid screening of a large number of samples.

### Preparation of the sample to be assayed

When it is desired to apply the technique developed in the present invention, a clinical isolate suspected of containing microorganisms possessing a gene coding for β-lactamase synthesis may be used directly or cultivated under conditions favorable to growth. After treating the targetted microorganisms to provide single stranded genomic nucleic acid and fixing the nucleic acid to a support, the affixed DNA is contacted with a labelled polynucleotide having a base sequence complementary to the coding or antisense strand of a gene coding for β-lactamase.

### Construction of the probes

The primary reagent is the labelled DNA probe. The probe of the present invention will normally have at least 12 bases and may have up to about 1700 bases. The probe sequence will be at least substantially complementary to a gene coding for production of β-lactamase. The probe does not need to have perfect complementarity to the sequence to which it hybridizes; there maybe 40% or more of mismatched pairs. Cross hybridization which broadens the specificity of the reaction, may be a result of variable reagents in the gene, mutation, a common probe for a plurality of microorganisms, alleles, or the like.

The probes used in the context of the present invention may be prepared by using the following general procedure: Escherichia coli strain HB101 is cultivated and purified and its plasmid DNA is isolated using the cleared lysate method and purified by cesium chloride-ethidium bromide gradiant ultracentrifugation.

Once the desired plasmid is isolated, it is then digested with the appropriate restriction endonucleases and alternatively ligated with pACYC184 which had been previously treated with the appropriate endonuclease in which case the resulting fragments are then treated with T4 ligase. Ligation is monitered by agarose gel electrophoresis. The recombinant plasmids thus obtained are transformed into E.coli HB101 for further growth. The bacterial clones which contain the recombinant plasmids are then chosen according to their resistance to certain antibiotics such as ampicillin and chloramphenicol. Chloramphenicol resistance is usually transfered to the bacterium by the pACYC184 plasmid. However, bacteria containing the recombinant plasmids become sensitive to tetracycline, because a given restriction site of pACYC184 is within the tetracycline gene and the foreign DNA inserted into this reagent destroys the gene.

From these experiments, the smaller recombinant plasmids are selected for further analysis. Once the desired plasmid is isolated and purified in sufficient amounts, it is digested with the appropriate restriction endonuclease and separated by electrophoresis. DNA fragments are excised from the gel and electroeluted in dialysis membranes. DNA is then precipitated in cold ethanol, centrifugated and dissolved in 100 µl of sterile TE. The purity and quality of each probe is then analyzed by agarose or acrylamide gel electrophoresis.

After having identified and characterized the specific DNA sequences responsible for β-lactamase production, corresponding DNA probes are synthesized. The oligonucleotide sequences are first synthesized using phosphite triester chemistry. The resulting sequences are then desalted, purified on polyacrylamide gels, visualized by ultraviolet shadowing and labelled.

For the most part, the polynucleotide probe will be labelled with a radioactive label. However, in some situations, it may be feasible to employ an antibody which will bind specifically to the probe hybridized to the single stranded DNA coding for β-lactamase synthesis. In this instance, the antibody would be labelled to allow for detection.

Among the types of radioactive labels that may be employed, there maybe mentionned $^{32}P$, $^{3}H$, $^{14}C$. However, any radioactive label which provides for an adequate signal and has sufficient half-life may be employed. Other labels include ligands, which can serve as a specific binding member to a labelled antibody, fluorescers, chemiluminescers, enzymes, antibodies which conserve as a specific binding pair member for a labelled ligand and the like. A wide variety of labels have been employed in immunoassays which can readily be employed with the probes of the present invention. The choice of the label will be governed by the effect of the label on the rate of hybridization and binding of the probe to the genetic DNA. It will be necessary that the label provides sufficient sensitivity to detect the amount of DNA available for hybridization.

The manner in which the label is bound to the probe will vary depending upon the nature of the label. For a radioactive label, a wide variety of techniques can be employed. Commonly employed is nick translation with an alpha-$^{32}P$-dNTP or terminal phosphate hydrolysis with alkaline-phosphatase followed by labelling with radioactive $^{32}P$ employing gamma-$^{32}P$-NTP and T4 polynucleotide kinase. Alternatively, nucleotides can be synthesized where one or more of the elements present are replaced with a radioactive isotope, e.g. hydrogen with tritium. The probes are now ready to be used for hybridization purposes.

## Hybridization technique

The probe is employed for hybridizing to a gene affixed to a water insoluble porous support where the single stranded nucleic acid is affixed. Depending upon the source of the nucleic acid, the manner in which the nucleic acid is affixed to the support may vary.

The sample containing the bacteria to be assayed may be spotted or spread onto a filter to provide a plurality of individual portions. The filter is an inert porous solid support such as nitrocellulose. The clinical isolate may be any excreta or physiological fluid such as urine, serum, plasma, or the like. The filter may be contacted with a nutrient source, to expend the numbers of the cells to form discrete colonies. The nutrients can diffuse to the cells, but the cells cannot diffuse away from their location on the filter. Conveniently, a microfilter is employed, which inhibits the passage of the cells through the filter and the filter placed on a nutrient gel.

The cells are then treated to liberate their DNA. If they were provided with nutrients to expand their numbers, after a sufficient time for the colonies to form, the filter is removed from the nutrient source and the cells lysed. Lysis conditions are devised such that the cells or colonies do not migrate and their DNA remains affixed in place on the surface where they were situated. For lysing, chemical lysing may be employed, usually dilute aqueous alkali. The alkali will also serve to denature the DNA. Other denaturation agents such as elevated temperatures, organic reagents, for example alcohols, amides, amines, ureas, phenols and sulfoxides or certain inorganic ions may be employed.

After denaturation, the filter is washed in a nucleus buffer solution, generally at pH of about 6 to 8. Of the many buffers that may be used, tris is an example. One or more washings may be involved, conveniently using the same procedure as employed for the lysing and denaturation.

After lysing, denaturing and washes have been accomplished, the DNA spotted filter is dried at an elevated temperature, generally from about 50 to 70 degrees Celsius. The DNA is now fixed in position and can be assayed with the probe when convenient.

Alternatively, plasmid DNA of the sampled bacteria may be isolated and purified before being spotted on the filter. In a subsequent step, the nucleic acids contained in the sample are rendered single stranded, and after having spotted the prepared mixture on nitrocellulose filters, the DNA spotted filter is dried at an elevated temperature. The DNA is now fixed in position and can be assayed with the probe when convenient.

Therefore, the previously prepared filter is then incubated at a mildly elevated temperature for a sufficient time with the hybridization solution without the probe to thoroughly wet the filter. Various hybridization solutions may be employed, comprising from about 20 to 60, preferably 30, percent of an inert polar organic solvent.

After the filter has been contacted with a hybridization solution at a moderate temperature for an extended period of time, the filter is then introduced into a second hybridization solution. The time for which the filter is maintained in the second solution may vary from 5 minutes to 3 hours or more. The second solution determines the stringency, dissolving cross duplexes and short complementary sequences. After rincing the filter at room temperature with an appropriate solution such as dilute sodium citrate-sodium chloride solution, the filter may now be assayed for the presence of duplexes in accordance with the nature of the label. In the event the label is radioactive, the filter may be dried and exposed to X-ray film.

Selection of the bacterial plasmids susceptible of carrying a β-lactamase gene

In order to render possible the synthesis of the DNA sequences which may be used for the construction of suitable β-lactam DNA probes, it is first necessary to identify and characterize the DNA sequences coding for β-lactamase production. Therefore, plasmids coding for a variety of β-lactamase types were first screened with a battery of restriction endonucleases to establish which produced the most convenient DNA fragments for cloning. For the cloning vector, vectors such as pBGS131, M13, pACYC184 and lacZ plasmid fusion vectors pMLB1034 and pMC1871 may be employed although pACYC184 is generally preferred. Thus, in the first step leading to the identification of the desired DNA sequences, plasmid DNA is digested with the appropriate endonucleases and ligated with the desired cloning vector. Endonucleases BamHI, EcoRI, HindIII, SalI, Sau3A and BlgII proved to be optimal when using plasmid pACYC184 as the cloning vector.

For example, plasmid DNA may be digested with BamHI, HindIII, SalI or Blg II and ligated with pACYC184 which had been previously treated with either the same or a different endonuclease, dephosphorylated with alkaline phosphatase, and the resulting mixture transformed into E. coli HB101 selecting for resistance to ampicillin and chloramphenicol. With plasmid Rms149 encoding for PSE-3, EcoRI plus SalI is used for cloning, and transformants resistant only to ampicillin are selected. For the AER-1 β-lactamase gene, Sau3A partial digest is prepared from chromosomal DNA of E. coli J53-2 07711 and combined with BamHI treated pACYC184. For plasmid R46 coding for the OXA-2 β-lactamase gene, digestion is effected using BlgII and the resulting fragment is ligated with pACYC184 which had been previously treated with BamHI. In the case of plasmid pMON31 coding for the SHV-1 β-lactamase gene, the plasmid is first digested with AvaI and the resulting fragment is ligated with T4 DNA ligase.

Once novel plasmid DNA has been isolated and purified, the smaller ampicillin recombinants are then selected for further investigation by construction of deleted plasmids. As expected from the location of the cloning sites within the tet gene of pACYC184, all recombinants proved to be tetracycline sensitive. pMON226 with an insert into the EcoRI site of pACYC184 was susceptible to chloramphenicol as well. Some plasmids also encoded resistance to streptomycin or sulfonamide in addition to ampicillin. Each of these plasmids was shown to produce the same β-lactamase type as the ampicillin resistant R46 parental plasmid by isoelectric focusing.

Restriction mapping and localisation of the β-lactamase structural gene

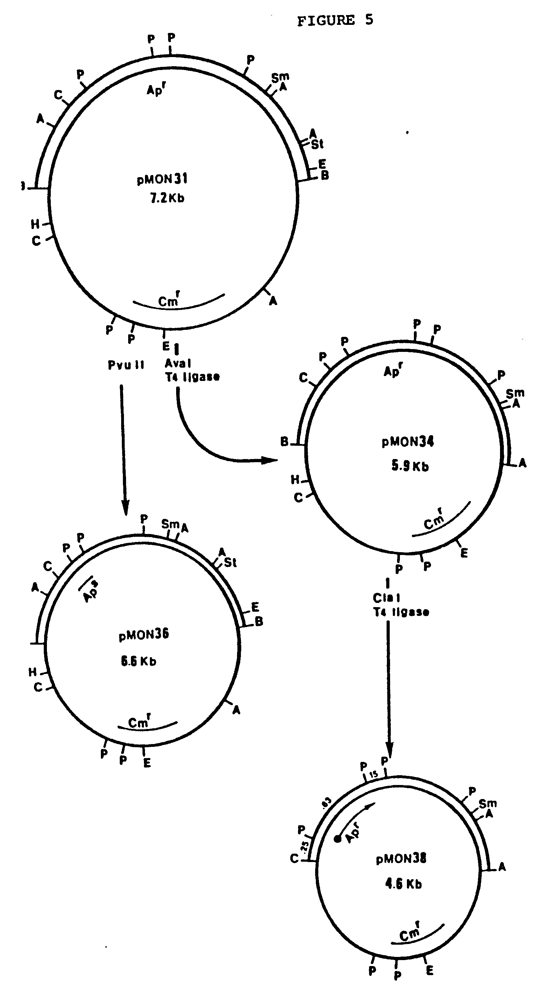

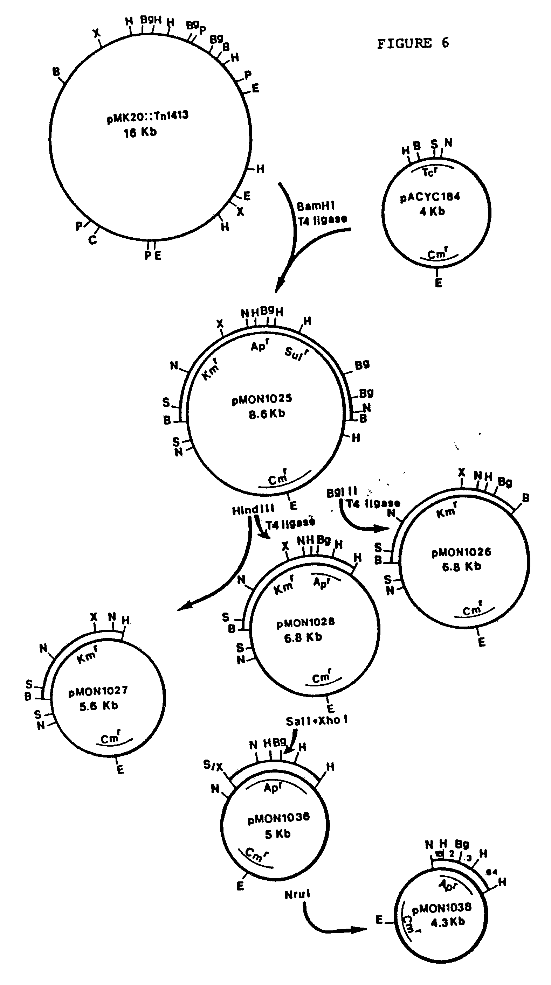

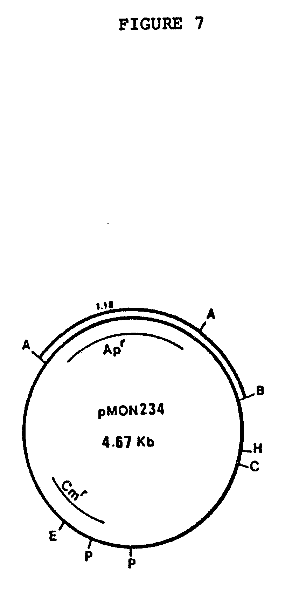

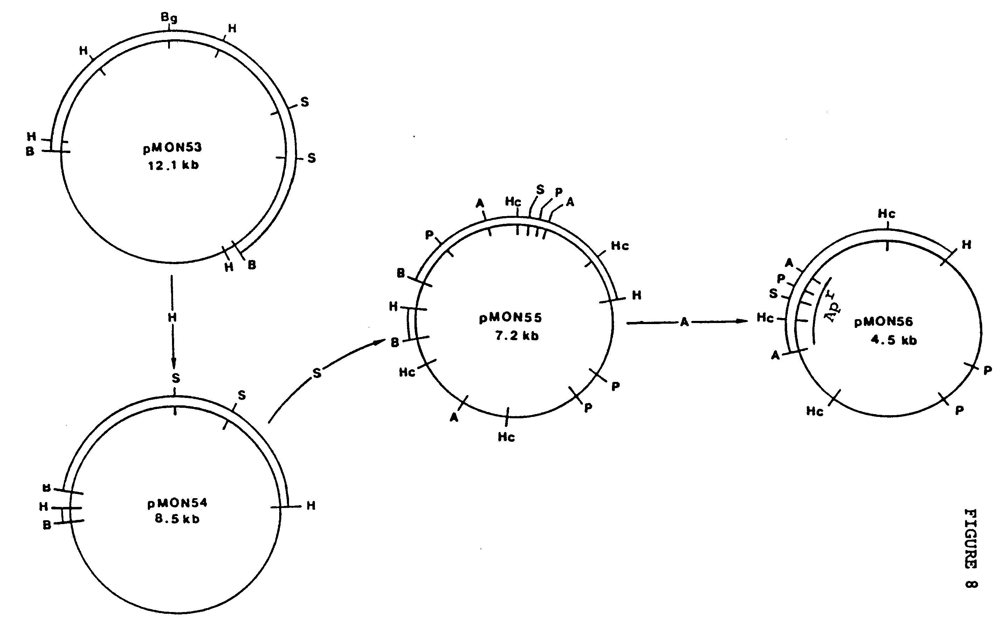

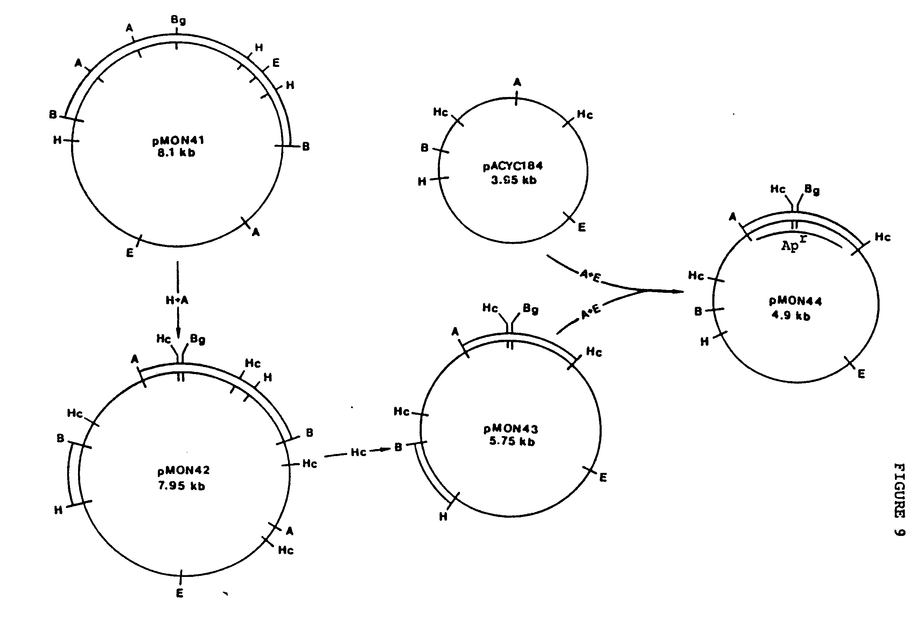

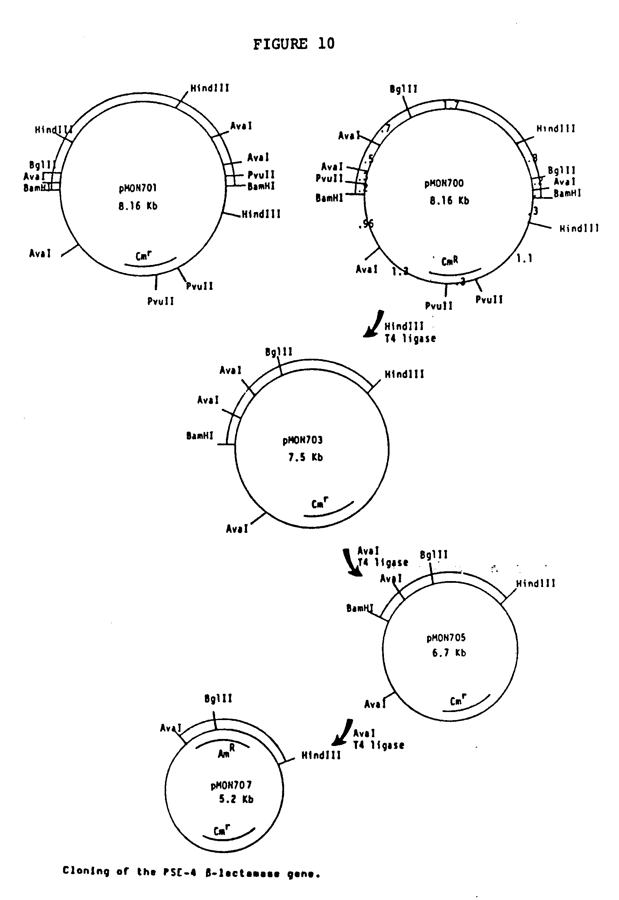

The DNA of the recombinant plasmids selected for further investigation may then be digested with the following endonucleases: AvaI, BamHI, BglII, EcoRI, HindIII, PvuII, SalI, SstI, and Sau3A, with the exception of plasmid pMON20 which may be digested using the enzymes AvaI, BamHI, EcoRI, HincII, HindIII, NruI, PstI, and SalI. The location of restriction sites is established by electrophoresis on agarose gels of 0.8 to 1.5% depending on fragment size using single, double, and triple digests in comparison with the corresponding restriction pattern of the cloning vector, such as pACYC184, or the initial plasmid, such as R46. Further localization of the β-lactamase structural genes is accomplished by subcloning or by deletion of fragments essential for gene expression. The resulting maps, shown in Figures 1, 2, and 5 to 10, revealed the following information: Figure 1 demonstrates that most β-lactamase genes are cloned on BamHI or HindIII fragments, and most of such fragments contain at least one BGlII site with the OXA-1 and OXA-4 β-lactamase fragments each containing two such sites. DNA fragments containing genes for the biochemically similar PSE-1, PSE-2, PSE-4, CARB-3, and AER-1 genes are also noteworthy for the frequency of similarly disposed AvaI and PvuII sites. Furthermore, it can be seen in Figure 2 that an additionnal PstI site has been mapped onto the sulfonamide gene of the R46 plasmid, which constitutes a discrepancy from the published R46 map. The OXA-2 β-lactamase structural gene is further localized by deleting a 2.8 kb PstI fragment and a 2.3 kb AvaI fragment. The deletion of these fragments yields plasmids pMON21 and pMON22. It is to be noted that the OXA-2 structural gene coding for β-lactamase resistance in the R46 plasmid has been found to be almost completely within the 0.85 kb AvaI-HincII-Hinc-II fragments except for the last few nucleotides.

Selection of DNA fragments and oligonucleotides used as β-lactamase DNA probes

From the restriction maps of Figure 1, 2 and 5 to 10, gene probes were developed for the TEM-1, OXA-1, PSE-1, ROB-1, OXA-2, OXA-3, OXA-4, ASF-2, PSE-4, CARB-3, CARB-4 and AER-1 β-lactamase genes, represented in Figures 1, and 3 to 10. The TEM-1 β-lactamase gene carried by plasmid pBR322 was sequenced in 1978 by Sutcliffe. Thus, a probe internal to the gene can be obtained as a 424 bp BGII plus HincII fragment. The second probe consists of a 315 bp BglII DNA fragment from pMON301 internal to the OXA-1 lactamase gene. It is to be noted that pMON301 is a BamHI-HindIII subclone of the HindIII fragment of pMON300 shown in Figure 1. Selection of this probe is based on the observation that deletion of this fragment eliminates β-lactamase gene expression. Furthermore, the promoter location and direction of transcription for the OXA-1 gene is established by inserting the 'lacZ cartridge from PMC1871 into the BGlII site of pMON301. The third probe is a 1.3 kb BamHI plus BGlII fragment containing part of the PSE-1 β-lactamase gene from pMON810. Its selection is based on the finding that insertion of the 'lacZ BamHI fragment from pMC1871 into the BglII site of pMON810 inactivates β-lactamase gene expression. A fourth probe consisting of a 250 bp Sau3A fragment from pMON401 is prepared based on the observation that deletion of this fragment eliminates β-lactamase gene expression. Probes internal to the OXA-2 structural gene are also prepared and included a 0.28 kb NruI-HincII and a 0.51 kb HincII-HincII DNA fragment. In order to confirm β-lactamase probe specificity, a 0.84 kb EcoRI-PstI fragment from plasmid pMON21 was also included since it was known to carry flanking sequences outside the structural gene.

In the β-lactamases of known sequence, the active site penicillin-binding serine residue is flanked by a phenylalanine at the fourth position and by a lysine at the third position on its carboxyl side, suggesting that these conserved phenylalanine and lysine residues are important for catalysis. In addition, it was noted that the TEM and OXA-2 β-lactamases also possessed an arg-X-X-X-arg in the same position. Hence, it was reasoned that OXA-2 and TEM oligonucleotide probes could be useful for estimating homology at or near β-lactamase active sites. From these nucleotide sequences, a series of oligonucleotides to be used as molecular probes were synthesized and are also shown in Figures 1, 3, 4 to 10. Table 1 summarizes the different β-lactamase gene probes synthesized.

TABLE 1.  SUMMARY OF β-LACTAMASE GENE PROBES

| Plasmid | β-lactamase | DNA Fragment Oligonucleotide | Cross hybridization |
|---|---|---|---|
| pBR322 | TEM-1 | 0.42 kb BglI-HincII<br>0.3 kb HincII-PstI<br>15-mer | TEM-1 TEM-2 TLE-1<br>TEM-1 TEM-2 TLE-1<br>TEM-1 TEM-2 |
| pMON301 | OXA-1 | 0.31 kb BGlII-BGlII<br>0.2 kb BglII-AvaI | OXA-1 OXA-4<br>OXA-1 OXA-4 |
| pMON21 | OXA-2 | 0.28 kb NruI-HincII<br>0.51 kb HincII-HincII<br>12-mer<br>15-mer 18-mer 45-mer | OXA-2<br>OXA-2 (OXA-3)<br>OXA-2 (OXA-3)<br>OXA-2 |
| pMON38 | SHV-1 | 0.78 kb PvuII-PvuII<br>17-mer | SHV-1 SHV-2<br>SHV-1 SHV-2 |
| pMON810 | PSE-1 | 1.3 kb BamHI-BGlII | PSE-1 PSE-4<br>CARB-3 (CARB-4) |
| pMON234 | PSE-2 | 1.2 kb AvaI-AvaI | PSE-2 (CARB-4) |
| pMON707 | PSE-4 | 1.7 Kb HindIII-BglII<br><br>0.7 kb BGlII-AvaI<br>0.7 kb BGlI-BSTeII | PSE-4 CARB-3<br>(PSE-2) (OXA-3)<br>PSE-4 CARB-3<br>PSE-4 (CARB-3)<br>(PSE-1) |
| pMON1028 | CARB-4 | 0.2 kb HindIII-BglII<br>0.3 kb BglII-HindIII<br>0.63 kb HindIII-HindIII<br><br>17-mer | CARB-4 (PSE-4)<br>CARB-4 (PSE-4)<br>CARB-4 (PSE-4)<br>(PSE-1)<br>CARB-4 |
| pMON510 | AER-1 | 0.41 kb AvaI-AvaI<br>0.34 kb AvaI-AvaI | AER-1<br>PSE-4 (OXA-1)<br>(OXA-4) |
| pMON401 | ROB-1 | 0.25 kb Sau3A-Sau3A | ROB-1 |
| pNU81 | ampC | 0.436 kb SalI-XhoI | CEP-1<br>(Chromosomal) |

Cross-hybridization in parenthesis indicates weak homology

7

The following examples are offered by way of illustration and not by way of limitation.

## EXAMPLE 1

### Preparation of $^{32}$P labelled 012 probe DNA

E.coli cells are grown in tryptic soy broth, purified, and its plasmid DNA is isolated by using the cleared lysate method and purified by cesium chloride-ethidium bromide gradiant ultracentrifugation. Purified R46 DNA plasmid is then digested with the restriction endonuclease BlgII and ligated with plasmid pACYC184 which had been previously treated with BamHI.

Ligation is monitored by electrophoresis on agarose gels of 0.8% and recombinant molecules are transformed into E.coli HB101 selecting for resistance to ampicillin and chloramphenicol. The resulting 10.3 kb pMON20 plasmid is then digested with the restriction endonuclease PstI and ligated using T4 DNA ligase to form the 7.5 kb pMON21 plasmid. Following purification of 20 μg of plasmid pMON20 or pMON21, probe DNA fragments are obtained from digestion of the pMON20 plasmid with AvaI plus HincII and from the digestion of pMON21 with AvaI plus HincII and separated by electrophoresis. These DNA fragments are separated in various concentrations of agarose (from 0.7% to 1.5%) or on 5% polyacrilamid gels and tris borate buffer. The DNA fragments are then excised from the gel and electroeluted in dialysis membranes. The DNA is then precipitated with cold ethanol, centrifugated and dissolved in 100 ul of sterile TE.(10 mM tris, 1 mM EDTA, ph 8.0). Among the fragments obtained, the desired probe had the following structure: -TCGACATTCAAG-. Once sufficient quantities of this oligonucleotide have been synthesized on an Applied Biosystem Apparatus or with the Pharmacia Gene Assembler using phosphite triester chemistry, the sequence is desalted using NAP10 columns, purified on 12% polyacrylamide gel containing 7M urea and visualized by ultraviolet shadowing. The phosphorylated oligonucleotide was labelled with T4 polynucleotide kinase and gamma-$^{32}$P-ATP at 37° C for 30 minutes.

## EXAMPLE 2

### Preparation of the $^{32}$P labelled O15 DNA probe

The procedure used to prepare $^{32}$P labelled O15 DNA probe was identical to the procedure described in Example 1. The 15 nucleotide sequence is demonstrated in Figure 4.

## EXAMPLE 3

### Preparation of the $^{32}$P labelled O18 DNA probe

The procedure used to prepare $^{32}$P labelled O18 DNA probe was identical to the procedure described in Example 1. The 18 nucleotide sequence is shown in Figure 4.

## EXAMPLE 4

### Preparation of $^{32}$P labelled O45 DNA probe

The procedure used to prepare $^{32}$P labelled O45 DNA probe was identical to the procedure described in Example 1. The 45 nucleotide sequence may be visualized in Figure 4.

## EXAMPLE 5

### Preparation of $^{32}$P labelled T15 DNA probe

The procedure used to prepare $^{32}$P labelled T15 DNA probe was identical to the procedure described in Example 1. The 15 nucleotide sequence is shown in Figure 4.

## EXAMPLE 6

### Preparation of $^{32}$P labelled TEM-1 probe DNA

E. coli cells are grown in tryptic soy broth, purified, and their plasmid DNA is isolated by using the cleared lysate method, concentrated with polyethylene glycol and purified by cesium chloride-ethidium bromide gradient ultracentrifugation. 100 μg of purified pBR322 plasmid is then digested with endonucleases BglI and HincII and the resulting DNA fragments are prepared by electrophoresis. These DNA fragments are separated in various concentrations of agarose (from 0.7% to 1.5%) or on 5% polyacrylamide gels and tris borate buffer. The DNA fragments are then excised from the gel and electroeluted in dialysis membranes. The DNA is then precipitated with cold ethanol, centrifugated and dissolved in 100 μl of sterile TE (10 mM tris, 1 mM EDTA, pH 8.0). Among the fragments obtained, the desired probe is demonstrated in Figure 3. Once sufficient quantities of this oligonucleotide have been synthesized on an Applied Biosystem Apparatus or with the Pharmacia Gene Assembler using phosphite triester chemistry, the sequence is desalted using NAP10 columns, purified on 12% polyacrylamide gel containing 7M urea and visualized by ultraviolet shadowing. Phosporylated oligonucleotide was then labelled with T4 polynucleotide kinase and gamma-$^{32}$P-ATP at 37° Celcius for 30 minutes.

EXAMPLE 7

Preparation of $^{32}$P labelled OXA-1 probe DNA

E. coli cells are grown in tryptic soy broth, purified, and their plasmid DNA is isolated by using the cleared lysate method, concentrated with polyethylene glycol and purified by cesium chloride-ethidium bromide gradient ultracentrifugation. Purified pMK20::Tn2603 DNA plasmid is then digested with the restriction endonuclease BamHI and ligated with plasmid pACYC184 which had ben previously treated with the same endonuclease and dephosphorylated with alkaline phosphatase. Ligation was monitored by electrophoresis on agarose gels of 8% and recombinant molecules were transformed into E. Coli HB101 selecting for resistance to ampicillin and chloramphenicol. The resulting 6.9 kb pMON300 plasmid was then digested with the restriction endonuclease HindIII and ligated using T4 DNA ligase to form the pMON301 plasmid. Following purification of 100 μg of plasmid pMON301, probe DNA fragments are obtained from digestion ot this plasmid with BglII and separated by electrophoresis. These DNA fragments are separated in various concentrations of agarose (from 0.7% to 1.5%) or on 5% polyacrylamide gels and tris borate buffer. The DNA fragments are then excised from the gel and electroeluted in dialysis membranes. The DNA was then precipitated with cold ethanol, centrifugated and dissolved in 100 μl of sterile TE (10 mM tris, 1 mM EDTA, ph 8.0). Among the fragments obtained, the desired probe has the structure shown in Figure 3. Once sufficient quantities of this oligonucleotide have been synthetized on an Applied Biosystem Apparatus or with the Pharmacia Gene Assembler using phosphite triester chemistry, the sequence was desalted using NAP10 columns, purified on 12% polyacrylamide gel containing 7M urea and visualized by ultraviolet shadowing. The phosporylated oligonucleotide was then labelled with T4 polynucleotide kinase and gamma-$^{32}$P-ATP at 37° C for 30 minutes.

EXAMPLE 8

Preparation of $^{32}$P labelled PSE-1 probe DNA

E. coli cells are grown in tryptic soy broth, purified, and their plasmid DNA is isolated by using the cleared lysate method, concentrated with polyethylene glycol and purified by cesium chloride-ethidium bromide gradient ultracentrifugation. Purified pMK20::TnI403 DNA plasmid is then digested with the restriction endonuclease BamHI and ligated with plasmid pACYC184 which had been previously treated with alkaline phosphatase. Ligation is monitered by electrophoresis on agarose gels of 0.8% and recombinant molecules are transformed into E. coli HB101 selecting for resistance to ampicillin and chloramphenicol. 100 μg of the purified pMON810 ou pMON811 plasmid are digested with BamII plus BglII ou BglII plus AvaI and the resulting fragments are separated by electrophoresis. These DNA fragments are separated in various concentrations of agarose (form 0.7% and 1.5%) or on 5% polyacrylamide gels and tris borate buffer. The DNA fragments were then excised from the gel and electroeluted in dialysis membranes. The DNA is then precipitated with cold ethanol after addition of 1/10 volume of 3M sodium acetate, ph 4.5. After centrifugation, the DNA pellet is resuspended in 500 ul of TE(10 mM tris, 1 mM EDTA, pH 8.0), proteins are extracted with chloroform, and the DNA is precipitated and dissolved in 100 μl of sterile TE. Among the fragments obtained, the desired probe has the structure demonstrated at Figure 3. Sequencing first once sufficient quantities of oligonucleotide have been synthesized on an Applied Biosystem Apparatus or with the Pharmacia Gene Assembler using phosphite triester chemistry, the sequence is desalted using NAP10 columns, purified on 12% polyacrylamide gel attaining 7M urea and visualized by ultraviolet shadowing. The phosporylated oligonucleotide was then labelled with T4 polynucleotide kinase and gamma-$^{32}$P-ATP at 32° C for 30 minutes.

EXAMPLE 9

9

Preparation of $^{32}$P labelled ROB 1 probe DNA

E.coli cells are grown in tryptic soy broth, purified, and their plasmid DNA is isolated by using the cleared lysate method, concentrated with polyethylene glycol and purified by cesium chloride-ethidium bromide gradient ultracentrifugation. Purified ROB161 DNA plasmid is then digested with the restriction endonuclease Sau3A and ligated with plasmid pACYC184 which had been previously treated with BamHI. Ligation was monitored by electrophoresis on agarose gels of 0.8% and recombinant molecules are transformed into E. coli HB101 selecting for resistance to ampicillin and chloramphenicol. 100 μg of the resulting 6.4 kb pMON401 plasmid are digested with the restriction endonuclease Sau3A and the DNA fragments obtained from that digestion are separated by electrophoresis. These DNA fragments are separated in various concentrations of agarose (from 0.6% to 1.5%) or on 5% polyacrylamide gels and tris borate buffer. The DNA fragments are then excised from the gel and electroeluted in dialysis membranes. The DNA is then precipitated with cold ethanol after addition of 1/10 volume of 3M sodium acetate, pH 4.5. After centrifugation, the DNA pellet is resuspended in 500 μl of TE (10 mM tris, 1 mM EDTA, pH 8.0), proteins are extracted with chloroform, and the DNA is precipitated and dissolved in 100 μl of sterile TE. Among the fragments obtained, the desired probe has the structure examplified in Figure 3. Sequence first once sufficient quantities of this oligonucleotide have been synthetized on an Applied Biosystem Apparatus or with the Pharmacia Gene Assembler using phosphite triester chemistry, the sequence is desalted using NAP10 columns, purified on 12% polyacrylamide gel containing 7M urea and visualized by ultraviolet shadowing. The phosphorylated oligonucleotide is then labelled with T4 polynucleotide kinase and gamma-$^{32}$P-ATP at 37° C for 30 minutes.

## EXAMPLE 10

### Hybridization techniques

Following the labelling of one μg of each DNA fragment to be used as a probe, each fragment is spotted on nitrocellulose filters using the Bi-Dot apparatus. The baked filters are then pre-hybridized for 4 to 8 hours at 42° C in 10 ml of 5 X SSC, 20% or 50% formamide, 5 X Denhardt's solution, 50mM phosphate buffer (pH 6.5), and 1 mg ml sonicated salmon sperm DNA.

The pre-hybridized solution is removed, and 10 ml of the same reagents plus denatured probe DNA ($10^5$cpm) is added.

The conditions selected to give a high degree of stringency are 42° C incubation for 18 hours with 50% formamide followed by washes of 65° C. Conditions for a low degree of stringency are 30° C incubation with 20% formamide followed by washes of 20° C. After the final wash, filter are mounted moist on Whatman 3mm paper, covered with plastic wrap, and audiographed at -70° C using Kodak X omat AR-2 film. Exposure varied from a few hours to several days. Positive and negative controls were included with each hybridization.

**Claims**

1. A method for assaying the presence of a gene coding for the synthesis of β-lactamase in a targetted microorganism, said method comprising:

a) depositing and fixing on an inert support a sample containing DNA fragments and substantially single-stranded form and suspected of coding for the synthesis of β-lactamase

b) contacting said fixed single stranded genetic material with a labelled probe having a nucleotide sequence of at least about 12 bases at least substantially complementary to a nucleotide sequence of a structural gene coding for the synthesis of β-lactamase, said contacting being under hybridizing conditions at a pre-determined stringency; and

c) Detecting duplex formation on said support by means of said label.

2. A hybrid recombinant plasmid capable of replication in an Escherichia Coli bacterial host species, said plasmid containing expressible heterologous DNA coding for the synthesis of β-lactamase.

3. A substantially pure DNA fragment having a molecular weight of 0.42 kb and coding for the synthesis of β -lactamase, said fragment being obtained from BglI-HincII digestion of pBR322 plasmid shown in Figure 3.

4. A substantially pure DNA fragment having a molecular weight of 0.3 kb and coding for the synthesis of β-lactamase, said fragment being obtained from HincII-PstI digestion of pBR322 plasmid shown in Figure 3.

5. A substantially pure DNA fragment having a molecular weight of 0.31 kb and coding for the synthesis of β -lactamase, said fragment being obtained from BglII-BglII digestion of pMON301 plasmid shown in Figure 3.

6. A substantially pure DNA fragment having a molecular weight of 0.2 kb and coding for the synthesis of β-lactamase, said fragment being obtained from BglII-AvaI digestion of pMON301 plasmid shown in Figure 3.

7. A substantially pure DNA fragment having a molecular weight of 0.28 and coding for the synthesis of β-lactamase, said fragment being obtained from NruI-HincII digestion of pMON21 plasmid shown in Figure 2.

8. A substantially pure DNA fragment having a molecular weight of 0.51 kb and coding for the synthesis of β-lactamase, said fragment being obtained from HincII-HincII digestion of pMON21 plasmid shown in Figure 2.

9. A substantially pure DNA fragment having a molecular weight of 0.78 kb and coding for the synthesis of β -lactamase, said fragment being obtained from PvuII-PvuII digestion of pMON38 plasmid shown in Figure 5.

10. A substantially pure DNA fragment having a molecular weight of 1.3 kb and coding for the synthesis of β-lactamase, said fragment being obtained from BamHI-BglII digestion of pMON810 plasmid shown in Figure 3.

11. A substantially pure DNA fragment having a molecular weight of 1.7 kb and coding for the synthesis of β-lactamase, said fragment being obtained from HindIII-BglII digestion of pMON707 plasmid shown in Figure 10.

12. A substantially pure DNA fragment having a molecular weight of 0.7 kb and coding for the synthesis of β-lactamase, said fragment being obtained from BglI-AvaI digestion of pMON707 plasmid shown in Figure 10.

13. A substantially pure DNA fragment having a molecular weight of 0.7 kb and coding for the synthesis of β-lactamase, said fragment being obtained from BglI-BstEII digestion of pMON707 plasmid shown in Figure 10.

14. A substantially pure DNA fragment having a molecular weight of 0.41 kb and coding for the synthesis of β -lactamase, said fragment being obtained from AvaI-AvaI digestion of pMON510 plasmid shown in Figure 1.

15. A substantially pure DNA fragment having a molecular weight of 0.34 kb and coding for the synthesis of β -lactamase, said fragment being obtained from AvaI-AvaI digestion of pMON510 plasmid shown in Figure 1.

16. A substantially pure DNA fragment having a molecular weight of 0.25 kb and coding for the synthesis of β -lactamase, said fragment being obtained from Sau3A-Sau3A digestion of pMON401 plasmid shown in Figure 1.

17. A substantially pure DNA fragment having a molecular weight of 4.67 kb and coding for the synthesis of β -lactamase, said fragment being obtained from AvaI-AvaI digestion of pMON234 plasmid shown in Figure 7.

18. A substantially pure DNA fragment having a molecular weight of 0.2 kb and coding for the synthesis of β-lactamase, said fragment being obtained from HindIII-BglII digestion of pMON1028 plasmid shown in Figure 6.

19. A substantially pure DNA fragment having a molecular weight of 0.3 kb and coding for the synthesis of β-lactamase, said fragment being obtained from BglI-HindIII digestion of pMON1028 plasmid shown in Figure 6.

20. A substantially pure DNA fragment having a molecular weight of 0.63 kb and coding for the synthesis of β-lactamase, said fragment being obtained from HindIII-HindIII digestion of PMON1028 shown in Figure 6.

21. A substantially pure single-stranded DNA fragment having an oligonucleotide sequence selected from : -TCGACATTCAAG-, -CGATCGAAGAAACGC-, -TCGAAGAAACGCTACTCG-, and -CGATCGAAGAAACGCTACTCGCCTGCATCGACATTCAAGATACCT-.

22. A hybridizable DNA fragment which comprises a strand which hybridizes with a complementary strand of any of the DNA fragments of any of claims 3 to 20 or with any of the single stranded fragments of claim 21 under pre-hybridization and subsequent hybridization conditions which comprise spotting said hybridizable DNA fragment on a nitrocellulose filter, baking the filters and then pre-hybridizing them for 4 to 8 hours at 42°C in 10 ml of 5 X SSC, 20 % or 50 % formamide, 5 X Denhardt's solution, 50 mM phosphate buffer (pH 6.5), and 1 mg/ml sonicated salmon sperm DNA, removing the pre-hybridized solution and adding denatured probe DNA, incubating for 18 hours at 42°C with a 50 % formamide solution and washing with said solution at 65°C.

23. A hybridizable DNA fragment which comprises a strand which hybridizes with a complementary strand of any of the DNA fragments of any of claims 3 to 20 or with any of the single stranded fragments of claim 21 under pre-hybridization and subsequent hybridization conditions which comprise spotting said hybridizable DNA fragment on a nitrocellulose filter, baking the filters and then pre-hybridizing them for 4 to 8 hours at 42°C in 10 ml of 5 X SSC, 20 % or 50 % formamide, 5 X Denhardt's solution, 50 mM phosphate buffer (pH 6.5), and 1 mg/ml sonicated salmon sperm DNA, removing the pre-hybridized solution and adding denatured probe DNA, incubating with a 20 % formamide solution and washing with said solution at 20°C.

# FIGURE 1

1 Kb

FIGURE 2

FIGURE 3

FIGURE 4

63                         77

-Arg- Ser- Lys - Lys- Arg- Tyr- Ser- Pro- Ala- Ser- Thr- Phe- Lys- Ile- Pro-

'5 — CGATCG AAG AAA CGC TACTCGCCTGCATCGACATTCAAGATACCT-3'    OXA—2

12-mer

15-mer

18-mer

45-mer

59                         73

-Arg- Pro- Glu- Glu- Arg -Phe -Pro- Met- Met- Ser- Thr- Phe- Lys- Val- Leu-

'5 — CGC CCC GAAGAACGTTTT CCAATGATG AGCACTTTTAAAGTTCTG-3'    TEM—1

15-mer

FIGURE 5

0300923

0300923

FIGURE 6

0300923

FIGURE 7

FIGURE 8

FIGURE 9

0300923

FIGURE 10

Cloning of the PSE-4 β-lactamase gene.